# EUROPEAN PATENT APPLICATION

(11) **EP 4 458 371 A1**
(43) Date of publication of application: **06.11.2024**
(21) Application number: 22916233.4
(22) Date of filing: 16.05.2022
(51) Int. Cl.: A61K 38/44, A61K 9/00, A61P 27/02, A61K 38/16

(54) **SUPEROXIDE DISMUTASE, AND USE THEREOF FOR PREVENTING OR TREATING DRY EYE SYNDROME**

(30) Priority: 27.12.2021 KR 20210189048; 15.04.2022 KR 20220047204
(71) Applicant: Genofocus, Inc., Daejeon 34014 (KR); BiomLogic, Inc., Seoul 03923 (KR)
(72) Inventor: PAN, Jae Gu, Seoul 03923 (KR); KIM, Eui Joong, Daejeon 34014 (KR); CHANG, In Ik, Seoul 03923 (KR); KIM, Jeong Hyun, Daejeon 34014 (KR)
(74) Representative: Hoefer & Partner Patentanwälte mbB
(86) International application number: PCT/KR2022/007003
(87) International publication number: WO 2023/128078

(57) **Abstract**

The present invention relates to a superoxide dismutase, and a use thereof for preventing or treating dry eye syndrome. More specifically, the present invention relates to a pharmaceutical composition or treatment method for preventing or treating dry eye syndrome. The composition and method according to the present invention bring about at least one among: increased tear secretion; increased tear film break-up time; the minimization of ocular surface damage; the protection of goblet cells in the conjunctiva; a reduction in the number of immune cells and inflammatory cytokines in the cornea and conjunctiva; reduced oxidative stress in the cornea, conjunctiva, and lacrimal gland; and reduced corneal epithelial apoptosis, and thus can be effectively used for preventing or treating dry eye syndrome.

## Description

### Technical Field

The present disclosure relates to a superoxide dismutase and a use thereof for preventing, ameliorating, or treating dry eye. More specifically, the present disclosure relates to a superoxide dismutase, a composition, or a method for preventing, ameliorating, or treating dry eye.

### Background Art

Dry eye is a major disease that is becoming increasingly common, affecting approximately 20% of adults in Korea. Recently, as the concept of dry eye has changed, dry eye has been defined as a multifactorial disease of the tear film and ocular surface which causes discomfort, visual impairment, tear film instability, and ocular surface damage, and is accompanied by increased tear osmotic pressure and ocular surface inflammation.

The pathogenesis of dry eye has not yet been clearly elucidated. However, several studies have reported that dry eye is related to inflammatory changes in ocular surface caused by tear hyperosmolarity, and various inflammatory factors contribute not only to the pathological process of dry eye but also to ocular discomfort. Patients with Sjögren's syndrome showed increased expression of inflammatory cytokines, such as IL-1, IL-6, IL-8, TNF-α, and IFN-γ, in tears and conjunctival epithelium, immunoreactive adhesion molecules, such as HLA-DR and ICAM-1, in conjunctival epithelial cells, and cytokines related to the Th-1 or Th-17 inflammatory pathway; and in addition, patients with dry eye showed increased concentration and activity of matrix metalloproteinase and increased apoptosis of the ocular surface epithelium. Thus, it was found that dry eye was directly related to inflammation of the ocular surface. Therefore, for treatment of dry eye, it is important to suppress inflammation of the ocular surface caused by tear hyperosmolarity.

Currently used treatments for dry eye include environmental control, artificial tears, anti-inflammatory treatments such as steroids or cyclosporine, tear secretion promoters, punctal occlusion, serum eye drops, therapeutic contact lenses, surgery and the like. The treatment is selected and used depending on severity of the disease; however, there is still no definitive treatment. Among these, combined administration of artificial tears and anti-inflammatory eye drops is the most widely used drug treatment for treating dry eye. The artificial tears can temporarily relieve symptoms of dry eye, but they cannot fundamentally treat inflammation on the ocular surface. Among the anti-inflammatory drugs, steroids have the disadvantage of causing serious complications such as glaucoma and cataracts. Thus, there is a need for a new treatment for dry eye.

Meanwhile, in 2006, Allergan developed, as a treatment for dry eye syndrome, Restasis Eye Drop that uses the immunomodulator cyclosporine. Restasis Eye Drop has been reported to inhibit production and activation of immune cells related to keratoconjunctivitis sicca and increase tear volume. However, since the agent exhibits medicinal efficacy through anti-inflammatory action, it needs to be repeatedly used for several months to obtain a satisfactory level of efficacy; and for the agent, there is a problem that burning sensation, which is a typical adverse effect, occurs at a high rate of about 17%.

Therefore, there is a need to develop an agent that is safe due to low incidence of adverse effects caused by eye-drop administration and can fundamentally prevent or treat dry eye syndrome, rather than a symptomatic treatment that simply relieves symptoms.

### Disclosure of Invention

### Technical Problem

The object of the present disclosure is to solve the above-mentioned problems of the prior art.

Another object of the present disclosure is to provide a superoxide dismutase for preventing, ameliorating, or treating dry eye.

Yet another object of the present disclosure is to provide a superoxide dismutase, which is derived from *Bacillus amyloliquefaciens* and for which oral administration efficacy and safety are secured, for preventing, ameliorating, or treating dry eye.

Still yet another object of the present disclosure is to provide a superoxide dismutase, which is derived from Bacillus or *Bacillus amyloliquefaciens* and is secreted extracellularly for convenient production, for preventing or treating dry eye.

Still yet another object of the present disclosure is to provide a pharmaceutical composition or treatment method for preventing or treating dry eye.

Still yet another object of the present disclosure is to provide a food or feed composition for preventing or ameliorating dry eye.

Still yet another object of the present disclosure is to provide a composition for oral administration for preventing, ameliorating, or treating dry eye, the composition comprising a superoxide dismutase.

The object of the present disclosure is not limited to the above-mentioned objects. The objects of the present disclosure will become clearer from the following description and may be realized by means and combinations thereof as set forth in the claims.

### Solution to Problem

Representative configurations of the present disclosure to achieve the above-mentioned objects are as follows.

According to an aspect of the present disclosure, there is provided an improved superoxide dismutase (SOD) for preventing, ameliorating, or treating dry eye.

According to another aspect of the present disclosure, there is provided a superoxide dismutase derived from a generally regarded as safe (GRAS) bacterium, such as Bacillus, for preventing, ameliorating, or treating dry eye.

According to still yet another aspect of the present disclosure, there is provided a superoxide dismutase, which is derived from Bacillus or *Bacillus amyloliquefaciens* and is secreted extracellularly for convenient production, for preventing, ameliorating, or treating dry eye.

According to still yet another aspect of the present disclosure, there is provided an improved superoxide dismutase that exhibits an effect of preventing, ameliorating, or treating dry eye even in a case of being administered orally.

According to still yet another aspect of the present disclosure, there is provided a pharmaceutical composition for preventing or treating dry eye, comprising the superoxide dismutase (SOD) as an active ingredient.

According to still yet another aspect of the present disclosure, there is provided a food or feed composition for ameliorating or preventing dry eye, comprising the superoxide dismutase as an active ingredient.

According to still yet another aspect of the present disclosure, there is provided a composition for oral administration for preventing, ameliorating, or treating dry eye, comprising the superoxide dismutase as an active ingredient.

According to still yet another aspect of the present disclosure, there is provided a method for preventing, ameliorating, or treating dry eye, comprising administering to a subject the superoxide dismutase or the composition.

According to still yet another aspect of the present disclosure, there is provided a use of the superoxide dismutase for preventing or treating dry eye.

According to still yet another aspect of the present disclosure, there is provided a use of the superoxide dismutase in manufacture of a medicament for prevention or treatment of dry eye.

### Advantageous Effects of Invention

According to the present disclosure, the superoxide dismutase, the composition, and the method can be effectively used to prevent, ameliorate, or treat dry eye through one or more selected from the effects of increasing tear volume, increasing tear film break-up time, minimizing ocular surface damage, protecting goblet cells in the conjunctiva, decreasing the number of immune cells and/or inflammatory cytokines in the cornea and/or conjunctiva, decreasing oxidative stress in the cornea, conjunctiva, and/or lacrimal gland, and decreasing corneal epithelial apoptosis. In particular, it was identified that the superoxide dismutase according to the present disclosure is effective in preventing, ameliorating, or treating dry eye even in a case of being administered orally.

The superoxide dismutase according to the present disclosure is derived from Bacillus or *Bacillus amyloliquefaciens* which is a generally regarded as safe (GRAS) bacterium. Thus, for the superoxide dismutase, not only can oral administration efficacy and safety be secured, but also production advantage can be taken by being directly recoverable from the supernatant during culture.

### Brief Description of Drawings

FIG. 1 illustrates a schematic diagram for the double crossover recombination performed in Example 1.1.
FIG. 2 illustrates results obtained by identifying defective genes using PCR in the expression host GFBS220 and the production strain BSBA310. W represents wild-type B. *subtilis* KCTC 3135, 220 represents the expression host GFBS220, and 310 represents the SodA2 production strain BSBA310.
FIG. 3 illustrates comparison of the amino acid sequences between SodA (Mn-SOD identified through N-terminal sequencing from the culture supernatant of *Bacillus amyloliquesfaciens* strain GF423) and the superoxide dismutase (SodA2) of the present disclosure.
FIG. 4 illustrates an expression vector for overexpression of *sodA2* gene. Here, rrnB T1T2 represents a transcription terminator; rep(pBR322) represents a pBR322-derived replicon that operates in *E. coli*.; rep(pUB110) represents a pUB110-derived replicon that operates in *B. subtilis;* Kan^{R} represents a kanamycin resistance gene (aminoglycoside O-nucleotidyltransferase); and BJ27 promoter represents a strong promoter for *B. subtilis.*
FIG. 5 illustrates an overall procedure for preparing the production strain BSBA310.
FIG. 6 illustrates results of the tear volume measurement performed in the examples of the present disclosure.
FIG. 7 illustrates results of the tear film break-up time (TBUT) measurement performed in the examples of the present disclosure.
FIG. 8 illustrates results of the corneal fluorescent staining score measurement performed in the examples of the present disclosure.
FIGS. 9A and 9B illustrate results of the measurement of the number of immune cells performed in the examples of the present disclosure.
FIGS. 10A, 10B, and 10C illustrate results of the reactive oxygen species measurement performed in the examples of the present disclosure.
FIGS. 11A and 11B illustrate results of the measurement of the number of goblet cells in the conjunctiva performed in the examples of the present disclosure.
FIGS. 12A and 12B illustrate results of the apoptosis measurement performed in the examples of the present disclosure.

### Best Mode for Carrying out Invention

The detailed description of the present disclosure set forth below will be described with reference to specific drawings with respect to specific embodiments in which the present disclosure may be practiced; however, the present disclosure is not limited thereto and, if properly described, is limited only by the appended claims, along with the full scope of equivalents to which such claims are entitled. It should be understood that various embodiments/examples of the present disclosure, although different, are not necessarily mutually exclusive. For example, a particular feature, structure, or characteristic described herein may be changed from one embodiment to another embodiment or implemented in combinations of embodiments without departing from the technical spirit and scope of the present disclosure. Unless otherwise indicated, terms used in describing the present disclosure are to be understood in their ordinary meaning and apply to the same terms used herein as well as to the aspect or embodiment of the disclosure for which the term is defined. For purposes of interpreting this specification, the following definitions will apply and whenever appropriate, terms used in the singular will also include the plural and vice versa.

### Definition

As used herein, the term "about" refers to a typical margin of error for each value known to those skill in the art.

As used herein, the term "subject" is used interchangeably with "patient," and may be any mammal in need of prevention or treatment of dry eye, such as primate (for example, human), companion animal (for example, dog, cat and the like), domestic animal (for example, cow, pig, horse, sheep, goat and the like), and laboratory animal (for example, rat, mouse, guinea pig and the like). In an embodiment of the present disclosure, the subject is a human.

The term "treatment" includes prophylactic and/or therapeutic treatment. The prophylactic and/or therapeutic treatment includes all types of treatment recognized in the art and includes, for example, administering the pharmaceutical composition or SOD of the present disclosure to a subject. If it is administered prior to clinical manifestation of a unwanted or undesirable condition (for example, disease or other unwanted or undesirable state in the subject), the treatment is prophylactic treatment (for example, it protects the subject against developing the unwanted or undesirable condition). On the other hand, if it is administered after clinical manifestation of a unwanted or undesirable condition, the treatment is therapeutic treatment, such as to diminish, ameliorate, or stabilize the existing unwanted or undesirable condition or side effects thereof.

The meaning of the term "prevention" is well known in the art. When used in the context of a medical condition, such as dry eye, it means reducing frequency or delaying onset and symptoms of a medical condition (for example, discomfort, visual impairment, tear film instability, ocular surface damage, increased tear osmotic pressure, ocular surface inflammation, and the like) in a subject who has received the pharmaceutical composition or SOD of the present disclosure, as compared with a subject who did not receive the same.

The term "administration" refers to providing an active ingredient to a subject to achieve a prophylactic or therapeutic purpose (for example, prevention or treatment of dry eye).

### Dry eye

The present disclosure is based, at least in part, on the discovery that administration, in particular, oral administration, of a superoxide dismutase (SOD) is effective in preventing or treating dry eye. Therefore, according to an aspect of the present disclosure, there is provided a use of an SOD for preventing, ameliorating, or treating dry eye.

The term "dry eye" is used interchangeably with dry eye syndrome or xerophthalmia, and refers to an eye disease that exhibits various symptoms, such as eye discomfort, dryness, foreign body sensation, itching, pain, and decreased vision, due to ocular surface damage resulting from a variety causes such as insufficient tears, excessive evaporation of the tear film, imbalance in tear composition, and inflammatory changes in tissues involved in the tear secretion process.

In addition, in the present disclosure, dry eye may be caused by other underlying diseases such as Sjögren's syndrome, or may be a complication caused by blepharitis or a foreign body in the eye. In addition, in the present disclosure, dry eye may be a result of infection, an adverse effect of medication, or a result of exposure to toxins, chemicals, or other substances. Therefore, in the present disclosure, dry eye may include both dry eye caused by Sjögren's syndrome or dry eye caused by non-Sjögren's syndrome.

The SOD according to the present disclosure may result in one or more selected from the group consisting of increased tear volume, increased tear film break-up time, minimized ocular surface damage, protection of goblet cells in the conjunctiva, decreased number of immune cells and/or inflammatory cytokines in the cornea and/or conjunctiva; decreased oxidative stress in the cornea, conjunctiva, and/or lacrimal gland; and decreased corneal epithelial apoptosis.

### Superoxide dismutase (SOD)

According to another aspect of the present disclosure, there is provided an improved superoxide dismutase (SOD) for preventing, ameliorating, or treating dry eye.

A superoxide dismutase (SOD) is an enzyme that alternately catalyzes dismutation of superoxide (O₂˙⁻) radical into either ordinary molecular oxygen (O₂) or hydrogen peroxide (H₂O₂). SODs play a key role in decreasing oxidative stress by removing reactive oxygen species. SODs are widely distributed in prokaryotic and eukaryotic cells and are classified into four classes based on their different types of metal centers (copper/zinc, nickel, manganese, and iron). Manganese-containing SODs (Mn-SODs) are widely present in many bacteria, chloroplasts, mitochondria, and cytosol of eukaryotic cells. The term "SOD" may be used interchangeably with a polypeptide having superoxide dismutase activity.

In an embodiment, the SOD may bind to manganese (Mn-SOD). Specifically, the SOD may be a deamidated Mn-SOD. More specifically, amino acid residues 73 and 136 of the SOD may be substituted with Asp residues, with respect to SEQ ID NO: 2. More specifically, the SOD may comprise or consist of the amino acid sequence represented by SEQ ID NO: 4.

The SOD or polypeptide having SOD activity of the present disclosure is interpreted to include amino acid sequences showing substantial identity to the above-mentioned amino acid sequence. The substantial identity means that in a case where aligned amino acid sequences are analyzed using an algorithm commonly used in the art, the sequences show sequence identity of 80% or higher, preferably 90% or higher, more preferably 95% or higher, and most preferably 98% or higher.

In another embodiment, the SOD may be a modified or engineered polypeptide having SOD enzymatic activity, and the polypeptide may comprise one or more mutations, for example, deletion, insertion, or substitution of one or more amino acids, which may or may not affect various aspects (in vivo, in vitro, or ex vivo stability, homogeneity, and/or conformational change). In addition, the polypeptide may further comprise a heterologous substance (for example, a tag known in the art, including HIS tag, HA tag, and myc tag, GFC, and/or Fc domain of an antibody) for purification, detection, or increased stability.

In an embodiment, the SOD may be derived from natural or recombinant microorganisms, and may be an enzyme produced through a process of isolation or purification from various sources.

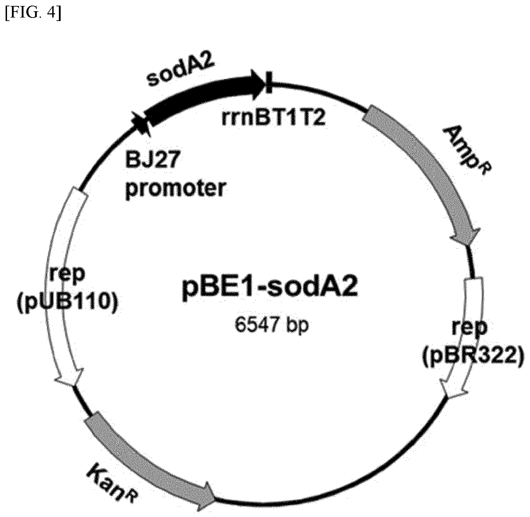

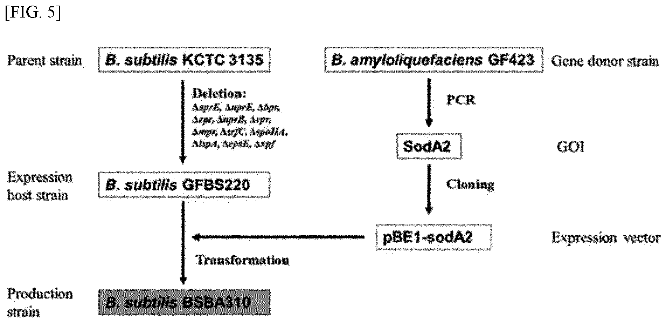

In an embodiment, the SOD may be derived from natural or recombinant microorganisms. For example, the SOD may be derived from bacteria. Preferably, the SOD may be derived from bacteria that are generally regarded as safe (GRAS) for use in drugs or foods. Specifically, the SOD may be derived from a Bacillus species strain. More specifically, the SOD may be derived from a *Bacillus amyloliquefaciens* strain. For example, the SOD may be derived from the *Bacillus amyloliquefaciens* strain GF423 (KCTC 13222 BP). The GF423 (KCTC 13222 BP) strain was deposited with the Korea Research Institute of Bioscience and Biotechnology on March 6, 2017. In addition, the SOD may be derived from a recombinant strain comprising the expression vector shown in FIG. 4. In addition, the SOD may be derived from a recombinant strain produced by a method including the method shown in FIG. 5. The recombinant strain may be a Bacillus species strain which comprises a nucleotide sequence encoding a polypeptide that comprises the amino acid sequence of SEQ ID NO: 4 and in which the following genes are deleted: *AprE, NprE, Bpr, Epr, NprB, Vpr, Mpr, IspA, SrfAC, spoIIAc, EpsE,* and *Xpf.* For detailed procedures for preparing the strain, see Example 1.

From the viewpoint that the SOD derived from the above-mentioned strain is an enzyme secreted extracellularly, in a case where an SOD is produced using the strain, it is possible to mass-produce an SOD, which is safe for humans, without going through an expensive purification process (for example, column purification), and this enables efficient production.

In an embodiment, the SOD may be isolated or purified from a variety of sources, including natural or recombinant hosts. For example, the SOD having activity of preventing or treating dry eye may be extracted from a culture supernatant of the *Bacillus amyloliquefaciens* strain GF423. Briefly, the Bacill*us amyloliquefaciens* strain GF423 may be first cultured in various types of media to obtain a culture. For example, the strain is grown at about 25°C to about 42°C for about 1 day to about 4 days using a complex medium (pH 6.0 to 7.0). Other suitable media for culturing the *Bacillus amyloliquefaciens* strain GF423 include Luria-Bertani (LB) medium, International Streptomyces Project (ISP) medium, nutrient agar (NA) medium, brain heart infusion agar (BHI) medium, sabouraud dextrose agar (SDA) medium, potato dextrose agar (PDA) medium, nutrient broth (NB) medium, and the like. Preferably, LB medium, ISP medium, BHI medium, SDA medium, or NB medium may be used. In addition, the SOD may be obtained from other natural or recombinant hosts using information provided in databases such as PubMed or BRENDA (www.brenda-enzymes.org).

In an embodiment, the SOD may be an isolated or purified enzyme. Here, the isolated or purified SOD, or a biologically active portion thereof is substantially free of cellular material or other contaminating proteins from the cell or tissue source from which it is derived. For example, the purified product may be separated purely from a strain culture through ultrafiltration, ammonium sulfate treatment, column purification, concentration, or the like, or may be a culture concentrate obtained therefrom through ultrafiltration, concentration, or the like. The phrase "substantially free of cellular material" includes preparations of a protein obtained by separating the protein from cellular components of the cell from which the protein is isolated or recombinantly produced. In an embodiment, the phrase "substantially free of cellular material" includes preparations of a protein in which unwanted proteins are present in an amount of less than about 30%, preferably less than about 20%, more preferably less than about 10%, and most preferably less than about 5% by dry weight.

In another embodiment, the SOD may be preferably purified by the following purification method, but the method is not limited thereto. The culture obtained by culturing the Bacillus amyloliquefaciens strain GF423 is centrifuged to collect a culture supernatant. The supernatant fraction is pretreated by solid-phase extraction, and then isolated and purified by chromatography. Here, various modes of chromatography may be used to purify the SOD. Preferably, hydrophobic interaction chromatography is used.

In another embodiment, the SOD may be included in a form of a strain lysate, strain culture, strain culture concentrate, or strain culture extract, or a dried form thereof. Here, the "strain lysate" may be a product obtained by culturing a strain and disrupting it mechanically or chemically, and may include any product obtained therefrom through additional processes such as extraction, dilution, concentration, and purification. The "strain culture" may refer to a culture itself obtained by culturing a strain or a supernatant thereof. The "strain culture concentrate" refers to a product that is separated purely from a strain culture through ultrafiltration, ammonium sulfate treatment, column purification, concentration, or the like, or a culture concentrate obtained therefrom through ultrafiltration, concentration, or the like. The "strain culture extract" refers to a product that is extracted from the culture or a concentrate thereof, and may include an extract, a diluted or concentrated solution of the extract, a dried product obtained by drying the extract, or a crude or purified product thereof, or a fraction obtained by fractionating the same. The dried form may include a lyophilized form.

In an embodiment, in a case of being administered orally to an individual in need of prevention or treatment of dry eye, the SOD of the present disclosure has exhibited one or more selected from the effects of increasing tear volume, increasing tear film break-up time, minimizing ocular surface damage, protecting goblet cells in the conjunctiva, decreasing the number of immune cells and/or inflammatory cytokines in the cornea and/or conjunctiva; decreasing oxidative stress in the cornea, conjunctiva; and/or lacrimal gland, and decreasing corneal epithelial apoptosis.

### Pharmaceutical composition

According to an aspect of the present disclosure, there is provided a pharmaceutical composition for treating or preventing dry eye, comprising a superoxide dismutase (SOD) as an active ingredient.

In an embodiment, the SOD according to the present disclosure may be combined with a pharmaceutically acceptable carrier, excipient and/or diluent to form a pharmaceutical composition for prevention or treatment of dry eye. The pharmaceutical composition of the present disclosure may be used interchangeably with the term veterinary composition when applied to an animal other than a human.

The pharmaceutical or veterinary composition of the present disclosure may further include one or more selected from the group consisting of pharmaceutically acceptable carriers, excipients, and diluents. The pharmaceutically acceptable carrier, excipient, and/or diluent may be those commonly used in the art. The carrier, excipient, or diluent may include, for example, lactose, dextrose, sucrose, sorbitol, mannitol, xylitol, erythritol, maltitol, starch, gum acacia, alginate, gelatin, calcium phosphate, calcium silicate, cellulose, methyl cellulose, hydroxypropyl methyl cellulose, microcrystalline cellulose, polyvinyl pyrrolidone, water, methyl hydroxybenzoate, propyl hydroxybenzoate, propyl hydroxybenzoate, talc, magnesium stearate, silicon dioxide, and mineral oil, but are not limited thereto.

In a case of performing preparation, the preparation may be achieved by using an additive such as a filler, an extender, a binder, a wetting agent, a disintegrant, or a surfactant. The additive for the preparation may be appropriately selected from those commonly used in the pharmaceutical field.

In addition, the pharmaceutical or veterinary composition of the present disclosure may be formulated into a desired form depending on the method of use, and in particular, the formulation may be achieved by employing a method known in the art to provide rapid, sustained, or delayed release of the active ingredient after being administered to a mammal. Specific examples of such a formulation include tablet, pill, powder, granule, syrup, solution, capsule, suspension, emulsion, injection solution, plaster, lotion, liniment, lemonade, aerosol, extract, elixir, ointment, fluid extract, needle, cream, soft or hard gelatin capsule, patch, and the like.

Furthermore, the pharmaceutical or veterinary composition of the present disclosure may preferably be formulated using an appropriate method known in the art or a method disclosed in Remington's Pharmaceutical Science (latest edition), Mack Publishing Company, Easton PA.

In an embodiment, the SOD may be administered orally or parenterally. In a case of being orally administered, the SOD may be coated with shellac for protection from gastric acid, but the coating agent is not limited thereto. Examples of the coating agent suitable for use in the present disclosure include shellac, ethyl cellulose, hydroxypropyl methylcellulose, hydroxypropyl methylcellulose phthalate, zein, Eudragit, and combinations thereof. In a case where the SOD is coated, the SOD may be coated in solution. Specifically, a purified solution and a shellac-containing solution are mixed and then lyophilized. This lyophilized sample may be made into powder and stored at about 4°C until use. Solid preparations for oral administration include tablets, pills, powders, granules, capsules and the like, and these solid preparations may be prepared by mixing a composition with at least one excipient, such as starch, calcium carbonate, sucrose, lactose, gelatin and the like. In addition to simple excipients, a lubricant such as magnesium stearate and talc may also be used. Examples of liquid preparations for oral administration include suspensions, oral solutions, emulsions, and syrups. In addition to commonly used simple diluents such as water and liquid paraffin, various types of excipients, such as a wetting agent, a sweetener, a fragrance, and a preservative, may be used.

For parenteral administration, the administration may be achieved by an injection method selected from sublingual administration, intraocular administration including intravitreal administration, nasal spray, topical dermal application, patch, intraperitoneal injection, intrarectal injection, subcutaneous injection, intravenous injection, intramuscular injection, or intrathoracic injection.

In addition, the pharmaceutical or veterinary composition of the present disclosure is administered in a pharmaceutically or veterinary effective amount. The term "pharmaceutically effective amount" or "veterinary effective amount" refers to an amount sufficient to treat a disease at a reasonable benefit/risk ratio applicable to any medical or veterinary treatment. A level of the effective amount may be determined depending on factors including the patient's or animal's body weight, gender, age, health status, severity of disease, activity of the drug, sensitivity to the drug, administration time, administration route and excretion rate, duration of treatment, and drugs used concurrently therewith, and other factors well known in the medical field.

The pharmaceutical or veterinary composition of the present disclosure may be administered as an individual therapeutic agent or in combination with other therapeutic agents, in which the administration may be carried out in a sequential or simultaneous manner. In addition, the pharmaceutical composition may be administered once or multiple times as needed. Taking all of the above factors into consideration, it is important to administer a minimum amount that allows the maximum effect to be obtained with no adverse effects, and such an amount may be easily determined by those skilled in the art.

### Prophylactic or therapeutic method

According to yet another aspect of the present disclosure, there is provided a method for treating or preventing dry eye, comprising administering, to a subject having or at risk of dry eye, the SOD or pharmaceutical composition according to the present disclosure.

In addition, there is provided a method for treating or preventing dry eye, comprising administering to a subject a superoxide dismutase. The details regarding the SOD, dry eye, treatment, or prevention are the same as described above regarding the pharmaceutical composition.

The term "administration" includes any of a variety of administrations that accomplish a desired action and enable treatment. The administration may include oral administration. For the oral administration, see the details described above for the pharmaceutical composition. The route of administration may be oral, parenteral, inhalation, topical or localized (for example, intralesional) administration. For example, parenteral administration may include, but is not limited to, intravenous, subcutaneous, intraperitoneal, intraarterial, and intraocular administration. In a case of being orally administered, as described above, the SOD may be administered in a form coated with a coating agent such as shellac.

An effective amount or effective non-toxic amount of the SOD according to the present disclosure may be determined by conventional experimentation. For example, a therapeutically active amount of the SOD of the present disclosure may vary depending on factors such as disease stage, disease severity, the subject's age, gender, medical comorbidities, and body weight. Dosage and dosing regimen of the SOD of the present disclosure may be adjusted to provide an optimal therapeutic response. For example, several divided doses may be administered daily, weekly, every two weeks, every three weeks, every four weeks and the like, and/or the dose may be proportionally reduced or increased depending on exigencies of therapeutic situation.

In an embodiment, the method may comprise administering one or more additional agents for treating or preventing dry eye. The one or more additional agents may be administered simultaneously, sequentially, or in reverse order with the SOD or pharmaceutical composition. In addition, the individual agents may be administered to the subject by the same or different routes.

### Food composition

According to still yet another aspect of the present disclosure, there is provided a food composition for ameliorating or preventing dry eye, comprising the SOD as an active ingredient. Such a food composition includes a medical or nutraceutical food composition.

The term "medical food" or "nutraceutical food" refers to a food manufactured from raw materials or ingredients that are likely to have beneficial functions in the human body, the food maintaining normal function or activating physiological functions of the human body to maintain or improve health. This food is defined by the Ministry of Food and Drug Safety, but is not limited thereto. The food does not exclude any common health food from its meaning.

In addition, the food includes, but is not limited to, various foods, food additives, beverages (for example, functional drinks, natural fruit juices, and vegetable drinks), gum, tea, vitamin complexes, health functional foods, other functional foods, and the like.

The food may be manufactured by conventional methods known in the art. For example, the medical food, nutraceutical food, or health functional food may be formulated into one selected from the group consisting of tablet, pill, powder, granule, capsule, and liquid formulation by further comprising at least one of a carrier, a diluent, an excipient, and an additive, in addition to the SOD, for the purpose of preventing or ameliorating dry eye. Specific examples of the carrier, excipient, diluent, and additive are well known in the art, and those skilled in the art are capable of combining appropriate ingredients depending on the formulation to manufacture the food.

An amount of the superoxide dismutase according to the present disclosure as an active ingredient in the above-described formulation may be adjusted appropriately depending on the form and purpose of use, the patient's condition, type and severity of symptoms, and the like. The amount may be, but is not limited to, 0.001 to 99.9% by weight, and preferably 0.01 to 50% by weight, based on the weight of solid content.

A dose of the food of the present disclosure may vary depending on the patient's age, body weight, gender, dosage form, health status, and severity of disease, and the administration may be done once a day or in divided doses several times a day at regular time intervals depending on the judgment of a doctor or pharmacist. For example, the daily dosage may be 10 to 1,000 mg/kg based on the active ingredient content. The dosage is an example of an average case and may increase or decrease depending on individual differences. In a case where the daily dosage of the health functional food of the present disclosure is less than the above-mentioned dosage, it is not possible to obtain significant effects. In a case where the daily dosage exceeds the above-mentioned dosage, it is not only uneconomical but also undesirable side effects may occur because such a dosage is outside the usual dosage range.

### Feed composition

According to still yet another aspect of the present disclosure, there is provided a feed composition for preventing or ameliorating dry eye, comprising the SOD according to the present disclosure. In the present disclosure, the food composition may include a feed composition. This feed composition of the present disclosure may be prepared in any formulation commonly used in the art. For example, the feed composition of the present disclosure may further comprise an auxiliary ingredient such as amino acids, inorganic salts, vitamins, antibiotics, antibacterial substances, antioxidants, antifungal enzymes, and other microbial agents in the form of live bacteria; grain such as ground or broken wheat, oats, barley, corn, and rice; vegetable protein feed such as those comprising rapeseed, soybean, and sunflower as main ingredients; animal protein feed such as blood meal, meat meal, bone meal, and fish meal; a dry ingredient consisting of sugar and a dairy product such as various powdered milk and whey powder; lipid such as animal fat and vegetable fat, optionally liquefied by heating; and an additive such as nutritional supplements, digestion and absorption enhancers, growth promoters, and disease prevention agents.

The feed composition of the present disclosure may be in a form of a powder or liquid preparation, and may comprise an excipient (for example, calcium carbonate, wheat shorts, zeolite, corn meal, rice bran, or the like) that is added to feed.

### Polynucleotide, expression vector, and host cell

According to still yet another aspect of the present disclosure, there is provided a polynucleotide encoding the superoxide dismutase polypeptide. Specifically, there is provided a nucleic acid that encodes a polypeptide comprising or consisting of the amino acid sequence of SEQ ID NO: 4.

According to still yet another aspect of the present disclosure, there is provided an expression vector comprising the nucleic acid. Specifically, the vector may be the expression vector shown in FIG. 4.

According to still yet another aspect of the present disclosure, there is provided a host cell comprising the nucleic acid or expression vector. Specifically, the host cell is a bacterial cell. More specifically, the host cell is a Bacillus species strain.

According to still yet another aspect of the present disclosure, there is provided a Bacillus species strain which comprises the polynucleotide sequence that encodes a polypeptide comprising the amino acid sequence of SEQ ID NO: 4 and in which the following genes are deleted: *AprE, NprE, Bpr, Epr, NprB, Vpr, Mpr, IspA, SrfAC, spoIIAc, EpsE, and Xpf.* For the detailed procedures for preparing the strain, see Example 1 below.

Hereinafter, the present disclosure will be described in more detail by way of the following examples. The following examples are presented to help understand the present disclosure. These examples are not intended to limit a scope of the present disclosure in any way and should not be construed as limiting the present disclosure.

### Examples

### Example 1. Preparation of recombinant strain expressing superoxide dismutase (SodA2)

### Example 1.1. Preparation of expression host GFBS220

The expression host GFBS220 was prepared using *Bacillus subtilis* KCTC 3135. *B. subtilis* KCTC 3135 was obtained from the Korea Research Institute of Bioscience and Biotechnology, Biological Resources Center (KCTC). To facilitate post-processing, the genes shown in Table 1 below were removed from the genome of *B. subtilis* KCTC 3135.

**[Table 1]**

| Gene name | Protein name | Function |
|---|---|---|
| *AprE* | Serine alkaline protease (subtilisin E) | Extracellular protease |
| *NprE* | Extracellular neutral metalloprotease | Extracellular protease |
| *Bpr* | Bacillopeptidase F | Extracellular protease |
| *Epr* | Extracellular serine protease | Extracellular protease |
| *NprB* | Extracellular neutral protease B | Extracellular protease |
| *Vpr* | Extracellular serine protease | Extracellular protease |
| *Mpr* | Extracellular metalloprotease | Extracellular protease |
| *IspA* | Intracellular serine protease | Intracellular protease |
| *SrfAC* | Surfactin synthase | Surfactin synthesis |
| *spoIIAC* | RNA polymerase sporulation-specific sigma factor (sigma-F) | Sporulation |
| *EpsE* | Putative glycosyltransferase | Extracellular polysaccharide |
| *Xpf* | RNA polymerase sigma factor | Transcription of PBSX prophage gene |

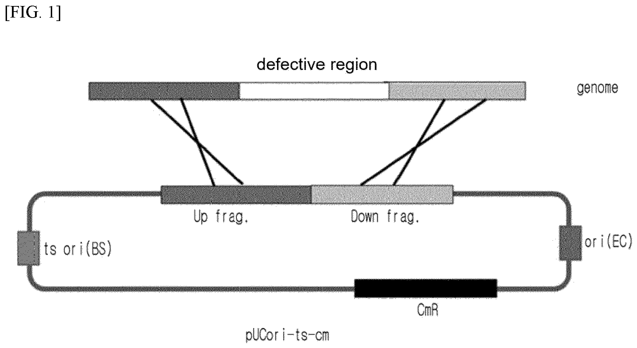

Removal of the genes was performed using double crossover recombination on the genome (FIG. 1). Specifically, DNA segments (up frag. and down frag.), which have homology to the DNA base sequences flanking the gene to be manipulated, were cloned into the vector pUCori-ts-cm that has a temperature-sensitive replication origin. PCR conditions and primers used for cloning are shown in Tables 2 and 3, respectively.

**[Table 2]**

| **PCR steps** | **Temperature** | **Time** |
|---|---|---|
| First denaturation | 95°C | 30 s |
| 30 cycles | 95°C | 30 s |
| | 50°C | 30 s |
| | 72°C | 1 min/kb |
| Final elongation | 72°C | 10 min |
| DNA polymerase used in PCR is *Taq* DNA polymerase (NEB, USA). | | |

**[Table 3]**

| Target gene | Homologous pair | | Primer sequence (5'→3') | SEQ ID NO |
|---|---|---|---|---|
| AprE | fragment 1 | F | ctacggggtctgacgcagatatcgtaaccgaagaacg | 5 |
| | | R | tgttgcagacatgttgctgaacgccatc | 6 |
| | fragment 2 | F | caacatgtctgcaacatatcttggaaac | 7 |
| | | R | gataagctgtcaaaccagagattggataggctatcaag | 8 |
| NprE | fragment 1 | F | ctacggggtctgacgcagggcattactgcaccataatc | 9 |
| | | R | gaggtacgccttcagcagcctgaacacc | 10 |
| | fragment 2 | F | gctgaaggcgtacctcacgccttcttcc | 11 |
| | | R | gataagctgtcaaaccagttgtcagatttgcatccttc | 12 |
| Bpr | fragment 1 | F | ctacggggtctgacgcagagcaatttccagcccgcttc | 13 |
| | | R | gtatgatgaggcatgaacagcaatcccg | 14 |
| | fragment 2 | F | tcatgcctcatcatactcaacaagggtg | 15 |
| | | R | gataagctgtcaaaccagttcccagccggatgttcaac | 16 |
| Epr | fragment 1 | F | ctacggggtctgacgcaggctttctgccagccgatagg | 17 |
| | | R | tgtgtcaaagccgttatgcttggctccg | 18 |
| | fragment 2 | F | taacggctttgacacagcacaaactgcc | 19 |
| | | R | gataagctgtcaaaccaggtctcccatacatgaacgac | 20 |
| NprB | fragment 1 | F | ctacggggtctgacgcagagtaatcttgtaggaggctg | 21 |
| | | R | tgtgactgtcatattcaccgtcgtgtgg | 22 |
| | fragment 2 | F | tgaatatgacagtcacacagtattccgcc | 23 |
| | | R | gataagctgtcaaaccagattaggatacggtctggctg | 24 |
| Vpr | fragment 1 | F | ctacggggtctgacgcagatgtacgctgagccgaatag | 25 |
| | | R | ggaatcacatttgcggagatacggcgtc | 26 |
| | fragment 2 | F | ccgcaaatgtgattccggcacatcaaac | 27 |
| | | R | gataagctgtcaaaccagaccgttttccgaatctgacc | 28 |
| Mpr | fragment 1 | F | ctacggggtctgacgcagcgttatcccgaatgcccgtg | 29 |
| | | R | ggctttgttcctttagtgtcaaccaccc | 30 |
| | fragment 2 | F | ctaaaggaacaaagccgattcgctccgc | 31 |
| | | R | gataagctgtcaaaccagaaattgactgctccacgctg | 32 |
| SrfAC | fragment 1 | F | ctacggggtctgacgcaggatatgtattacctatcgcc | 33 |
| | | R | gccccttcatccagttcactgcttccttg | 34 |
| | fragment 2 | F | ggaagcagtgaactggatgaaggggcttcgctg | 35 |
| | | R | gataagctgtcaaaccagaacgcttcgacatcactttc | 36 |
| spoIIA | fragment 1 | F | ctacggggtctgacgcagagcggacgatgggagactgg | 37 |
| | | R | gccacctcatgcagccgatctggaagag | 38 |
| | fragment 2 | F | ggctgcatgaggtggctgagcggctcgg | 39 |
| | | R | gataagctgtcaaaccagctcattgttttggtgagctg | 40 |
| IspA | fragment 1 | F | ctacggggtctgacgcagtgctgcttggcattcatttc | 41 |
| | | R | gccattgaagcaatgcctccgtttgaatc | 42 |
| | fragment 2 | F | acggaggcattgcttcaatggctgcccctcatg | 43 |
| | | R | gataagctgtcaaaccagtcaatgctctcgtcatattc | 44 |
| EpsE | fragment 1 | F | ctacggggtctgacgcagcaagcaaatgggctgggagg | 45 |
| | | R | gacaagccatgctttctgccaaagtgcg | 46 |
| | fragment 2 | F | gaaagcatggcttgtcaagcatgaatag | 47 |
| | | R | gataagctgtcaaaccagtgttgtatacattcagcagc | 48 |
| Xpf | fragment 1 | F | gatcctctacggttcatccatgtccgaac | 49 |
| | | R | tctatgatcctcctcatttttg | 50 |
| | fragment 2 | F | gaggaggatcatagaaagcttgtcatacgtttgcc | 51 |
| | | R | gagttttcgttcggatcctccgctttttgtttg | 52 |

The thus produced vectors were introduced into *B. subtilis* cells, and then transformants (single crossover homologous recombination) were selected using medium containing chloramphenicol at 37°C (non-replication temperature). The selected transformants were cultured (second crossover) at 30°C (replication-permissive temperature) in LB medium without antibiotics, and then cultured at 39°C in LB agar medium without antibiotics. Colonies susceptible to chloramphenicol were selected from the resulting colonies (plasmid curing). The selected colonies were subjected to PCR to obtain double crossover recombinants, and second pure separation was performed to select the final recombinant, which was named GFBS220.

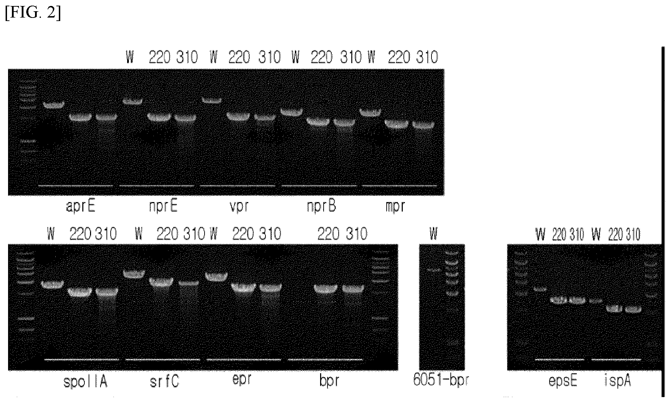

The defective gene regions of GFBS220 were amplified with the primers shown in Table 4 and PCR, and then identified using agarose gel electrophoresis. The results obtained by identifying the defective genes using PCR in the expression host GFBS220 are shown in FIG. 2.

**[Table 4]**

| Gene region (locus) | PCR primer (SEQ ID NO) | Size of PCR product of *B. subtilis* KCTC 3135 | Size of PCR product of GFBSL210 |
|---|---|---|---|
| *AprE* | F caaggcttgaaataacgttg (SEQ ID NO: 53) | 3115bp | 2116bp |
| | R ttcgctgattacaacattgg (SEQ ID NO: 54) | | |
| *NprE* | F ggaacagatgatagctcatc (SEQ ID NO: 55) | 3487bp | 2145bp |
| | R acactccgagaaggctgaag (SEQ ID NO: 56) | | |
| *Vpr* | F ctgcggcctgcacagccgcc (SEQ ID NO: 57) | 3733bp | 2293bp |
| | R cgctgaatgacggtggtaag (SEQ ID NO: 58) | | |
| *NprB* | F agtaatcttgtaggaggctg (SEQ ID NO: 59) | 2666bp | 2153bp |
| | R cgccaatgaagtgaaggagg (SEQ ID NO: 60) | | |
| *Mpr* | F ggttgaggcgattgcgacgg (SEQ ID NO: 61) | 2734bp | 2076bp |
| | R ccattctgcaaaatcagctc (SEQ ID NO: 62) | | |
| *spoiIIAC* | F aagatatgaagaaagccggg (SEQ ID NO: 63) | 2628bp | 2102bp |
| | R acagccgcttcataagcctg (SEQ ID NO: 64) | | |
| *ThrC* | F tcaagctgtcatgtacggag (SEQ ID NO: 65) | 376bp | 5455bp |
| | R tccgatacgaatggctgtcg (SEQ ID NO: 66) | | |

### Example 1.2. Construction of expression vector pBE1-sodA2

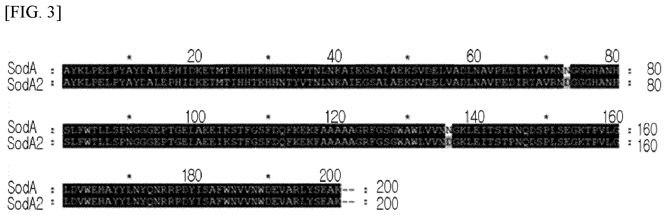

An Mn-SOD was identified in the culture supernatant of *Bacillus amyloliquesfaciens* strain GF423 through N-terminal sequencing (Kang et al., 2018). The GF423 strain was deposited with the Korea Research Institute of Bioscience and Biotechnology on March 6, 2017 (under accession number of KCTC 13222 BP). The gene of the Mn-SOD was named *sodA* according to the nomenclature for bacterial SOD. For the amino acid sequence thereof, see FIG. 3 and SEQ ID NO: 2 (for the nucleotide sequence thereof, see SEQ ID NO: 1). LC-UV-MS/MS peptide mapping of the enzyme preparation produced 100% amino acid sequence coverage; however, deamidation was observed at Asn73 and Asn136 residues at 73% and 17% abundance, respectively. Therefore, to improve homogeneity of the purified enzyme, both Asn residues were replaced with Asp residues. For the amino acid sequence of such variant *sodA,* named SodA2, see FIG. 3 and SEQ ID NO: 4 (for the nucleotide sequence thereof, see SEQ ID NO: 3).

The *sodA2* gene was amplified with duplicate elongation PCR using four primers (Table 5) and the genome of *Bacillus amyloliquefaciens* GF423 as a template. Nucleotides for replacement of Asn with Asp are underlined.

**[Table 5]**

| Primer | | Sequence (5'→3') | SEQ ID NO |
|---|---|---|---|
| 1 | SOD F | GGAGGAATTACAATGGCTTA CAA | 67 |
| 2 | D74 R | TGCATGTCCGCCGCCATCGT TGCGGAC | 68 |
| 3 | D74 F | CAGTCCGCAACGATGGCGGC GGACATG | 69 |
| 4 | D137 R | TTCAAGTTTGCCGTCGTTTAC AACGAGC | 70 |
| 5 | D137 F | CTCGTTGTAAACGACGGCAA ACTTG | 71 |
| 6 | SOD R | CAAAACAGAAGCTTCATTAT TTTGCT | 72 |

The amplified DNA fragment containing the *sodA2* gene and the plasmid pBE1 linearized by treatment with NdeI and HindIII were assembled *in vitro* by the SLIC method (Jeong et al. 2012), and then the resulting construct was transformed into *E. coli* C2984H. Correct clones were screened by restriction enzyme mapping and identified by nucleotide sequencing. The resulting expression vector pBE1-sodA2 is shown in FIG. 4.

### Example 1.3. Preparation of production strain BSBA310

The expression vector pBE1-sodA2 was transformed into *B. subtilis* GFBS220. Strains were selected from the transformants, and pure clones were established by two single colony isolation procedures in LB2 medium. The selected strain was named BSBA310 and stored at -80°C using the glycerol stock method. The results obtained by identifying the defective genes using PCR in this production strain BSBA310 are shown in FIG. 2. In addition, the overall procedure for preparing the production strain BSBA310 is summarized in FIG. 5.

### Example 2. Isolation and purification of superoxide dismutase (SodA2) from production strain BSBA310

### Example 2.1. Culture of production strain BSBA310

For culture of the production strain BSBA310 obtained in Example 1, the single colony formed on LB agar medium (LB (Luria-Bertani) agar; 10 g/L of tryptophan, 5 g/L of yeast extract, 10 g/L of NaCl, 15 g/L of agar) was inoculated into 30 ml of LB medium and cultured at 37°C for 12 hours. This seed culture was again inoculated into 3 L of LB medium containing 1 mM manganese sulfate (MnSO₄), and cultured at 37°C for 20 hours.

### Example 2.2. Isolation and purification of SodA2

The cell culture obtained in Example 2.1 was centrifuged at 4°C and 3,578 x g for 20 minutes to collect the supernatant, which was then concentrated 10-fold using ultrafiltration (hereinafter referred to as UF, MWCO 10,000). 390 g of ammonium sulfate was added thereto per liter of the concentrated cell culture, the mixture was stirred for 20 minutes, and centrifugation was performed to take the supernatant. The taken supernatant was purified using a phenyl Sepharose HP column. The purification process was as follows. The column was equilibrated using 50 mM potassium phosphate pH 7.0 with a concentration of 2 M ammonium sulfate. Then, the supernatant obtained by adding ammonium sulfate was passed through the column, and SodA2 attached to the column was recovered using 50 mM potassium phosphate pH 7.0 containing 1.6 M ammonium sulfate. The solution purified through the column was collected and concentrated by ultrafiltration while removing highly concentrated salts formed during the purification process. The concentrate was filtered through a sterilization filter and then lyophilized. Activity of SodA2 was analyzed using the SOD assay kit (Cayman Chemical, Michigan, USA). One unit of SOD activity is defined as an amount of the enzyme that inhibits superoxide radicals by 50%. Activity of the dried SodA2 enzyme ranged from 1000 to 1200 U/mg.

### Example 3. Preparation of material to be administered

Shellac (EXCELACS Co., Ltd., Bangkok) was dissolved to a concentration of 3% ethanol and sterilized using a 0.2 µm sterile filter. Shellac dissolved in ethanol was diluted with PBS.
Lyophilized SodA2 was dissolved at 20 mg/ml, and the shellac solution and the SOD solution were mixed in a 1:1 ratio, and then the mixture was lyophilized. The lyophilized shellac-coated SodA2 was mixed with dextrin at a ratio of 1:9 to 12 (shellac-coated SodA2:dextrin) to prepare a material for use in animal tests. SOD activity of the final material was set to be 90 to 110 U/mg.

### Example 4. Identification of therapeutic or prophylactic effect on dry eye

In this example, analysis was conducted in an experimental dry eye (EDE)-induced C57BL/6 mouse model to identify effects of oral SodA2 at concentrations of 2.5 mg/kg, 5 mg/kg, and 10 mg/kg on tear volume, tear film break-up time, ocular surface damage, conjunctival goblet cells, inflammatory cytokines on the ocular surface, CD4+ T immune cells, oxidative stress and reactive oxygen species, and corneal epithelial apoptosis.

### Example 4.1. Preparation of animal model with experimental dry eye (EDE)

To induce experimental dry eye, 7- to 8-week-old C57BL/6 mice (purchased from Damool Science) were injected subcutaneously with scopolamine hydrochloride at 0.5 mg/0.2 ml three times a day under exposure to a ventilating device for 18 hours a day. The humidity was maintained at 30%, and the temperature was maintained at 25°C. The C57BL/6 mice were classified into 7 groups, depending on concentrations of the oral drug to be administered, as shown in Table 6.

**[Table 6]**

| Grouping | |
|---|---|
| UT | Untreated control (normal), n = 6 |
| EDE | EDE only (EDE control), n = 6 |
| Vehicle | EDE + vehicle (negative control), administered once a day, n = 6 |
| 0.05% CsA | EDE + 0.05% CsA (positive control), administered in eye drop form twice a day, n = 6 |
| 2.5 mg/kg oral SodA2 | EDE + oral SodA2 2.5 mg/kg, administered once a day, n = 6 |
| 5 mg/kg oral SodA2 | EDE + oral SodA2 5 mg/kg, administered once a day, n = 6 |
| 10 mg/kg oral SodA2 | EDE + oral SodA2 10 mg/kg, administered once a day, n = 6 |
| Here, CsA refers to cyclosporin A. | |

### Example 4.2. Drug administration and test procedures

After inducing experimental dry eye in the experimental mice, the drugs prepared in Example 3 were orally administered at 2 µL per dose once a day to the treatment groups (administered at 13:30 every day). In both eyes of all mice, at baseline, day 5, and day 10, tear volume and tear film break-up time were measured and corneal fluorescent staining score was examined. The mice were then sacrificed and tissue analysis was performed. Each group consisted of 6 animals.

### Measurement of tear volume

Tear volume was determined as follows. A phenol red-impregnated cotton thread was brought into contact for 20 seconds with the conjunctival sac on the lateral canthus side of each mouse, and the length was measured using SMZ microscopy. Then, the measured length was converted into a volume by substituting it into a given formula (see Villareal AL, Farley W, Pflugfelder SC. Effect of topical ophthalmic epinastine and olopatadine on tear volume in mice. Eye Contact Lens. 2006; 32:272-6).

### Measurement of tear film break-up time

To evaluate tear film stability, which is known to be the most important clinical factor in dry eye, tear film break-up time was measured. 1 µl of 1% fluorescent dye (fluorescein) was instilled into the lower conjunctival sac of each mouse, and then each mouse was allowed to blink about three times. Subsequently, the eyes were gently made open, and then the time (seconds) until the first defect occurred in the stained tear film was measured using a slit lamp microscope equipped with a cobalt blue filter. This was repeated three times to obtain the average value.

### Measurement of corneal fluorescent staining score

1 µL of 1% fluorescent dye was instilled into the eyes of each mouse, followed by washing with saline. Subsequently, the cornea of each mouse was observed using a slit lamp microscope to score the degree of epithelial injury (the extent to which fluorescent staining occurred). To this end, the cornea was divided into four zones and the respective zones were evaluated from 0 to 4 points as shown in Table 7, and then the points were summed up (a total of 0 to 16 points) (see Pauly A, Brignole-Baudouin F, Labbe A, Liang H, Warnet JM, Baudouin C: New tools for the evaluation of toxic ocular surface changes in the rat. Invest Ophthalmol Vis Sci. 2007; 48:5473-83).

**[Table 7]**

| | |
|---|---|
| 0 | No fluorescein staining |
| 1 | Slightly punctuate staining <30 spots |
| 2 | Punctuate staining <30 spots, but not diffuse |
| 3 | Severe diffuse staining but no positive plaque |
| 4 | Severe diffuse staining with positive fluorescein plaque |

### Measurement of malondialdehyde (MDA) through ELISA

The corneas, conjunctivas, and lacrimal glands were excised from both eyes (6 eyes) of 3 animals in each group required for tissue analysis. The samples were homogenized in PBS, and then the lysates were centrifuged at 10000 x g for 10 minutes at 4°C. The supernatant was taken and the total amount of protein was measured using the Bradford assay. MDA was detected using an ELISA (Enzyme-linked immunosorbent assay) kit (manufactured by Quantikine, R&D Systems, Minneapolis, MN) containing capture and detection antibodies according to the manufacturer's manual. Using ELISA, MDA levels in the cornea, conjunctiva, and lacrimal gland were measured and recorded, respectively.

### Measurement of inflammatory cytokines using Luminex multiplex immunobead assay

Conjunctival tissue was excised and collected from 6 eyes of 3 animals in each group, and then immersed in lysis buffer containing protease inhibitors for 30 minutes. Subsequently, the resultant was centrifuged and stored -80°C. Then, the concentrations of IFN-γ, IL-1β, and TNF-α in the cornea and conjunctiva were analyzed using the Luminex multiplex immunobead assay.

### Measurement of number of immune cells and reactive oxygen species (DCF-DA) through flow cytometry

The cornea, conjunctiva, and lacrimal gland were excised, sectioned with scissors, and then shaken with 0.5 mg/mL of collagenase type D (manufactured by Roche Applied Science, Indianapolis, IN, USA) at 37°C for 60 minutes. The tissue was ground and then allowed to pass through a cell strainer, followed by centrifugation. The number of CD4+IFN-γ+ T cells was calculated by performing flow cytometry with fluorescein-conjugated anti-CD4 antibody and phycoerythrin-conjugated anti-IFN-γ antibody, and the number of DCF-DA cells was calculated using the CM-H2DCFDA kit (manufactured by Thermo Fisher Scientific, Waltham, MA, USA).

### Histology

The eye and accessory tissues were excised from one eye of one animal in each group, fixed in 4% paraformaldehyde, embedded in paraffin, and then PAS staining was performed on two cross-sectional pieces of the tissue. In each piece, the density of goblet cells in the conjunctiva was calculated using a microscope and image analysis software.

### Immunofluorescent staining

The eye and accessory tissues were excised from one eye of one animal in each group, fixed in 4% paraformaldehyde, embedded in paraffin, and then TUNEL staining was performed on two cross-sectional pieces of the tissue. In each piece, the degree of apoptosis in the conjunctiva was identified using a microscope (Leica TCS SP5 AOBS laser scanning confocal microscope (Zeiss GmbH)) and image analysis software (NIS Elements version 4.1; Nikon, Melville, NY, USA).

### Statistical analysis

SPSS 22.0 was used for analysis, and the results for continuous variables were expressed as mean ± SD. Normality was identified by the Shapiro-Wilk test. Comparison between groups was analyzed post hoc using one-way analysis of variance (ANOVA) followed by Fishers LSD post hoc test. P-value of less than 0.05 was considered statistically significant.

### Example 4.3. Results

### Tear volume

Before drug administration, the tear volume was 0.046 ± 0.004 µL in the UT group, 0.023 ± 0.004 µL in the EDE group, 0.024 ± 0.003 µL in the vehicle group, 0.023 ± 0.003 µL in the 0.05% cyclosporine A (CsA) group, 0.024 ± 0.003 µL in the 2.5 mg/kg oral SodA2 group, 0.022 ± 0.004 µL in the 5 mg/kg oral SodA2 group, and 0.023 ± 0.004 µL in the 10 mg/kg oral SodA2 group.

On day 5 of administration, the tear volume was 0.045 ± 0.006 µL in the UT group, 0.02 ± 0.003 µL in the EDE group, 0.021 ± 0.003 µL in the vehicle group, 0.026 ± 0.004 µL in the 0.05% CsA group, 0.026 ± 0.005 µL in the 2.5 mg/kg oral SodA2 group, 0.026 ± 0.005 µL in the 5 mg/kg oral SodA2 group, and 0.026 ± 0.005 µL in the 10 mg/kg oral SodA2 group.

On day 10 of administration, the tear volume was 0.043 ± 0.005 µL in the UT group, 0.017 ± 0.003 µL in the EDE group, 0.018 ± 0.003 µL in the vehicle group, 0.027 ± 0.005 µL in the 0.05% CsA group, 0.027 ± 0.005 µL in the 2.5 mg/kg oral SodA2 group, 0.03 ± 0.005 µL in the 5 mg/kg oral SodA2 group, and 0.028 ± 0.004 µL in the 10 mg/kg oral SodA2 group.

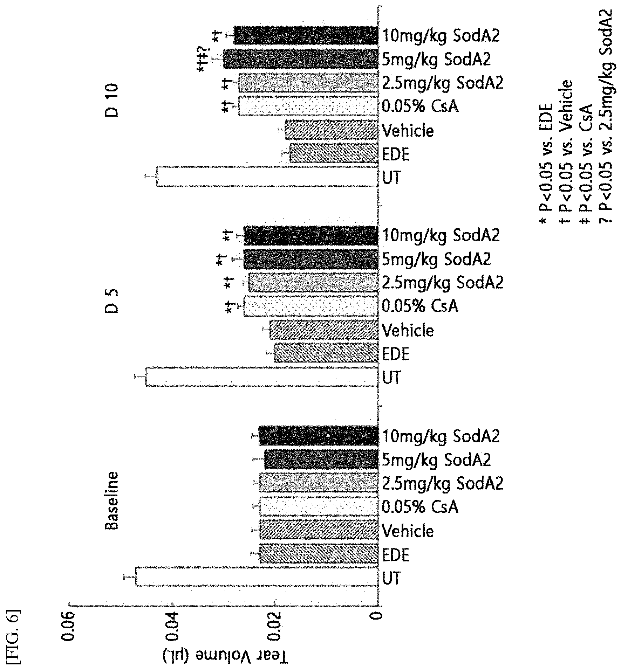

On days 5 and 10 of administration, there was a significant increase in tear volume in all of the 0.05% CsA group, the 2.5 mg/kg oral SodA2 group, the 5 mg/kg oral SodA2 group, and the 10 mg/kg oral SodA2 group, as compared with the EDE and vehicle groups. In particular, the 5 mg/kg oral SodA2 group showed a more significant increase than the 0.05% CsA group. These results obtained by measuring tear volume are shown in FIG. 6.

### Tear film break-up time

Before drug administration, the tear film break-up time was 2 ± 0.45 seconds in the UT group, 1.43 ± 0.19 seconds in the EDE group, 1.43 ± 0.23 seconds in the vehicle group, 1.46 ± 0.13 seconds in the 0.05% CsA group, 1.44 ± 0.15 seconds in the 2.5 mg/kg oral SodA2 group, 1.42 ± 0.16 seconds in the 5 mg/kg oral SodA2 group, and 1.41 ± 0.25 seconds in the 10 mg/kg oral SodA2 group.

On day 5 of administration, the tear film break-up time was 2.21 ± 0.4 seconds in the UT group, 1.02 ± 0.2 seconds in the EDE group, 1.06 ± 0.23 seconds in the vehicle group, 1.3 ± 0.2 seconds in the 0.05% CsA group, 1.23 ± 0.2 seconds in the 2.5 mg/kg oral SodA2 group, 1.31 ± 0.3 seconds in the 5 mg/kg oral SodA2 group, and 1.28 ± 0.3 seconds in the 10 mg/kg oral SodA2 group.

On day 10 of administration, the tear film break-up time was 2.1 ± 0.4 seconds in the UT group, 0.91 ± 0.2 seconds in the EDE group, 0.93 ± 0.2 seconds in the vehicle group, 1.39 ± 0.2 seconds in the 0.05% CsA group, 1.21 ± 0.3 seconds in the 2.5 mg/kg oral SodA2 group, 1.28 ± 0.3 seconds in the 5 mg/kg oral SodA2 group, and 1.3 ± 0.3 seconds in the 10 mg/kg oral SodA2 group.

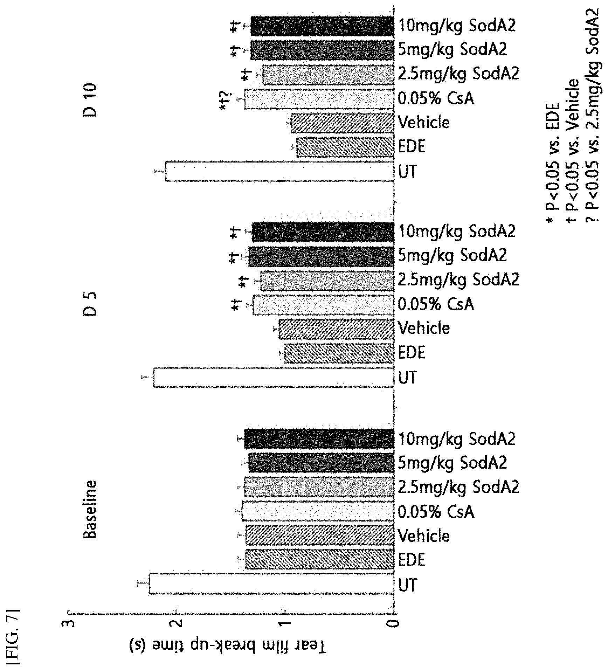

On days 5 and 10 of administration, there was a significant increase in tear film break-up time in all of the 0.05% CsA group, the 2.5 mg/kg oral SodA2 group, the 5 mg/kg oral SodA2 group, and the 10 mg/kg oral SodA2 group, as compared with the EDE and vehicle groups. In particular, the 5 mg/kg oral SodA2 group and the 10 mg/kg oral SodA2 group showed an increased tear film break-up time similar to the 0.05% CsA group. These results obtained by measuring tear film break-up time are shown in FIG. 7.

### Corneal fluorescent staining score

Before drug administration, the corneal fluorescent staining score was 1.8 ± 1.1 points in the UT group, 9.5 ± 1.8 points in the EDE group, 9.1 ± 2 points in the vehicle group, 9.1 ± 2 points in the 0.05% CsA group, 9.2 ± 1.7 points in the 2.5 mg/kg oral SodA2 group, 9.4 ± 1.7 points in the 5 mg/kg oral SodA2 group, and 9.3 ± 1.7 points in the 10 mg/kg oral SodA2 group.

On day 5 of administration, the corneal fluorescent staining score was 2.9 ± 1.4 points in the UT group, 12.1 ± 2.1 points in the EDE group, 11.7 ± 2.2 points in the vehicle group, 10.4 ± 2.5 points in the 0.05% CsA group, 10.4 ± 1.9 points in the 2.5 mg/kg oral SodA2 group, 9.9 ± 2.5 points in the 5 mg/kg oral SodA2 group, and 10.1 ± 2.9 points in the 10 mg/kg oral SodA2 group.

On day 10 of administration, the corneal fluorescent staining score was 3.6 ± 1.4 points in the UT group, 14.1 ± 1.8 points in the EDE group, 13.5 ± 1.6 points in the vehicle group, 8.9 ± 2 points in the 0.05% CsA group, 8.8 ± 2 points in the 2.5 mg/kg oral SodA2 group, 7.8 ± 1.9 points in the 5 mg/kg oral SodA2 group, and 8.2 ± 1.9 points in the 10 mg/kg oral SodA2 group.

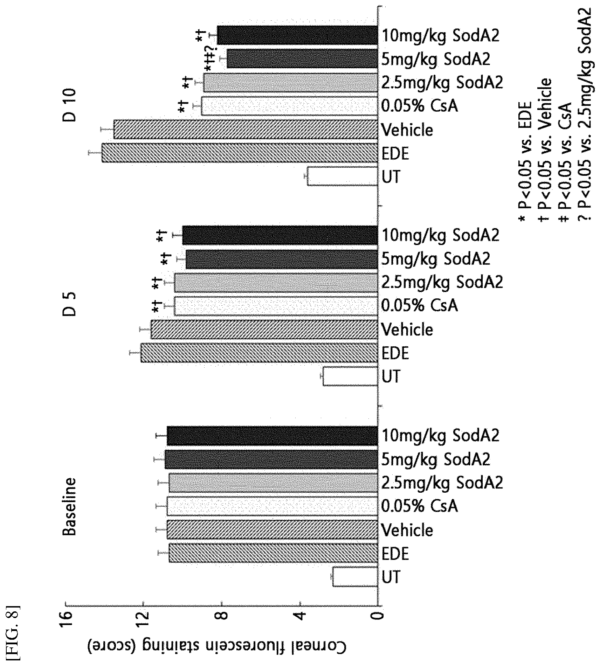

On days 5 and 10 of administration, there was a significant decrease in corneal fluorescent staining score in all of the 0.05% CsA group, the 2.5 mg/kg oral SodA2 group, the 5 mg/kg oral SodA2 group, and the 10 mg/kg oral SodA2 group, as compared with the EDE and vehicle groups. In particular, the 5 mg/kg oral SodA2 group showed a more significant decrease than the 0.05% CsA group. These results obtained by measuring corneal fluorescent staining score are shown in FIG. 8.

### Malondialdehyde (MDA) concentration

The results obtained by measuring the MDA concentration in the cornea, conjunctiva, and lacrimal gland are presented in Table 8. The unit is in pmol/ml.

**[Table 8]**

| | **Cornea** | **Conjunctiva** | **Lacrimal gland** |
|---|---|---|---|
| **UT** | 12.1 ± 3.16 | 17.8 ± 12.2 | 14.6 ± 7.25 |
| **EDE** | 30.9 ± 21.1 | 28.0 ± 8.92 | 31.0 ± 13.6 |
| **Vehicle** | 27.7 ± 27.4 | 26.1 ± 4.64 | 37.0 ± 12.6 |
| **0.05% CsA** | 17.7 ± 9.62 ^{†} | 19.1 ± 7.99 ^{†} | 22.2 ± 11.4 ^{†} |
| **2.5 mg/kg oral SodA2** | 22.7 ± 13.8 ^{†} | 21.5 ± 9.26 ^{†} | 22.6 ± 7,72 ^{†} |
| **5 mg/kg oral SodA2** | 20.2 ± 14.0 ^{†} | 19.0 ± 7.17 ^{†} | 18.8 ± 11.6 ^{†} |
| **10 mg/kg oral SodA2** | 18.5 ± 10,6 ^{†} | 16.3 ± 9.12 ^{†} | 22.5 ± 11.4 ^{†} |

As can be seen in Table 8, the groups treated with CsA and oral SodA2 showed a decreased MDA concentration in both the cornea, conjunctiva, and lacrimal gland, as compared with the EDE and vehicle groups. In particular, 10 mg/kg oral SodA2 showed similar (cornea, lacrimal gland) or superior (conjunctiva) efficacy to 0.05% CsA.

### Inflammatory cytokines

The results obtained by measuring inflammatory cytokines in the cornea and conjunctiva are presented in Table 9. The unit is in pg/ml.

**[Table 9]**

| | | **IFN-γ** | **IL-1β** | **TNF-α** |
|---|---|---|---|---|
| **Cornea** | **UT** | 9.28 ± 1.28 | 14.26 ± 6.09 | 14.6 ± 7.25 |
| | **EDE** | 18.81 ± 18.32 | 23.12 ± 12.09 | 31.0 ± 13.6 |
| | **Vehicle** | 17.90 ± 16.02 | 22.90 ± 9.91 | 37.0 ± 12.6 |
| | **0.05% CsA** | 11.95 ± 8.27 | 19.98 ± 8.14 | 22.2 ± 11.4 |
| | **2.5 mg/kg oral SodA** | 10.77 ± 8.30 | 15, 45 ± 8.93 | 22.6 ± 7.72 |
| | **5 mg/kg oral SodA2** | 9.80 ± 6.11 | 13.17 ± 2.26 | 18.8 ± 11.6 |
| | **10 mg/kg oral SodA2** | 7.89 ± 7.90 | 8.69 ± 4.18 | 22.5 ± 11.4 |
| **Conjunctiva** | **UT** | 12.98 ± 5.14 | 17.8 ± 12.2 | 14.6 ± 7.25 |
| | **EDE** | 35.96 ± 25.62 | 28.0 ± 8.92 | 31.0 ± 13.6 |
| | **Vehicle** | 37,42 ± 26.15 | 26.1 ± 4.64 | 37.0 ± 12.6 |
| | **0.05% CsA** | 25.33 ± 13.71 | 19.1 ± 7.99 | 22.2 ± 11.4 |
| | **2.5 mg/kg oral SodA** | 21.27 ± 17.49 | 21.5 ± 9.26 | 22.6 ± 7.72 |
| | **5 mg/kg oral SodA2** | 18.28 ± 11.75 | 19.0 ± 7.17 | 18.8 ± 11.6^{†} |
| | **10 mg/kg oral SodA2** | 15.53 ± 11.43 | 16.3 ± 9.12 | 22.5 ± 11.4 |

As can be seen in Table 9, the groups treated with CsA and oral SodA2 showed a tendency of decreased concentrations of IFN-γ, IL-1β, and TNF-α in the cornea and conjunctiva, as compared with the EDE and vehicle groups. In particular, oral SodA2 at all doses tended to decrease inflammatory cytokines more than CsA.

### Measurement of number of immune cells (CD4+IFN-γ T cells) through flow cytometry

The proportion of CD4+IFN-γ+ T cells in the cornea was 16.53 ± 1.65% in the UT group, 48.39 ± 1.34% in the EDE group, 47.02 ± 0.04% in the vehicle group, 26.05 ± 0.18% in the 0.05% CsA group, 21.18 ± 0.15% in the 2.5 mg/kg oral SodA2 group, 23.87 ± 0.17% in the 5 mg/kg oral SodA2 group, and 30.24 ± 1.33% in the 10 mg/kg oral SodA2 group.

Meanwhile, the proportion of CD4+IFN-γ+ T cells in the conjunctiva was 17.40 ± 3.43% in the UT group, 46.63 ± 1.27% in the EDE group, 43.65 ± 2.68% in the vehicle group, 22.67 ± 0.47% in the 0.05% CsA group, 21.00.± 0.63% in the 2.5 mg/kg oral SodA2 group, 22.87 ± 2.47% in the 5 mg/kg oral SodA2 group, and 25.83 ± 0.27% in the 10 mg/kg oral SodA2 group.

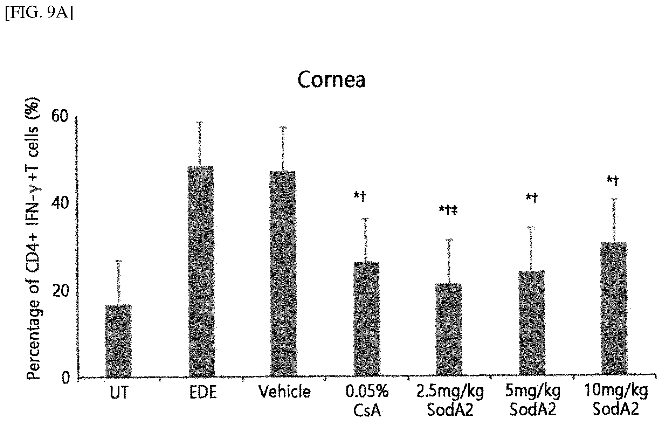

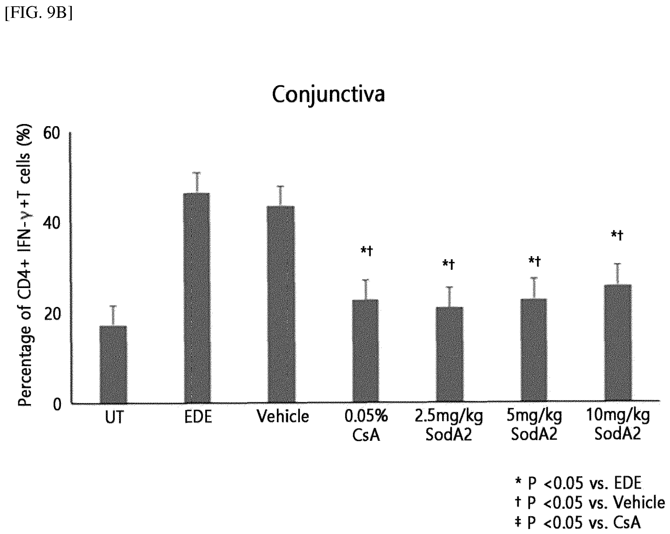

In the cornea and conjunctiva, the CsA and oral SodA2 groups showed a significant decrease in proportion of CD4+IFN-γ+ T cells as compared with the EDE and vehicle groups. In particular, in the cornea, the 2.5 mg/kg oral SodA2 group showed a significantly lower proportion of CD4+IFN-γ+ T cells than the 0.05% CsA group. These results obtained by analyzing the number of CD4+IFN-γ+ T cells in the cornea and conjunctiva are shown in FIGS. 9A and 9B, respectively.

### Measurement of reactive oxygen species

The amount of reactive oxygen species generated in the cornea due to dry eye was 33.93 ± 24.62% in the UT group, 87.43 ± 49.83% in the EDE group, 76.25 ± 49.54% in the vehicle group, 50.27 ± 11.27% in the 0.05% CsA group, 33.57 ± 26.51% in the 2.5 mg/kg oral SodA2 group, 54.53 ± 13.53% in the 5 mg/kg oral SodA2 group, and 57.38 ± 23.43% in the 10 mg/kg oral SodA2 group.

In the conjunctiva, the amount was 42.25 ± 23.23% in the UT group, 97.69 ± 54.19% in the EDE group, 95.48 ± 54.54% in the vehicle group, 58.16 ± 5.54% in the 0.05% CsA group, 55.13 ± 7.41% in the 2.5 mg/kg oral SodA2 group, 57.63 ± 8.16% in the 5 mg/kg oral SodA2 group, and 53.29 ± 1.19% in the 10 mg/kg oral SodA2 group.

Meanwhile, in the lacrimal gland, the amount was 36.88 ± 26.91% in the UT group, 100.90 ± 80.95% in the EDE group, 114.26 ± 65.19% in the vehicle group, 58.66 ± 11.11% in the 0.05% CsA group, 59.05 ± 10.77% in the 2.5 mg/kg oral SodA2 group, 56.03 ± 6.37% in the 5 mg/kg oral SodA2 group, and 49.92 ± 4.19% in the 10 mg/kg oral SodA2 group.

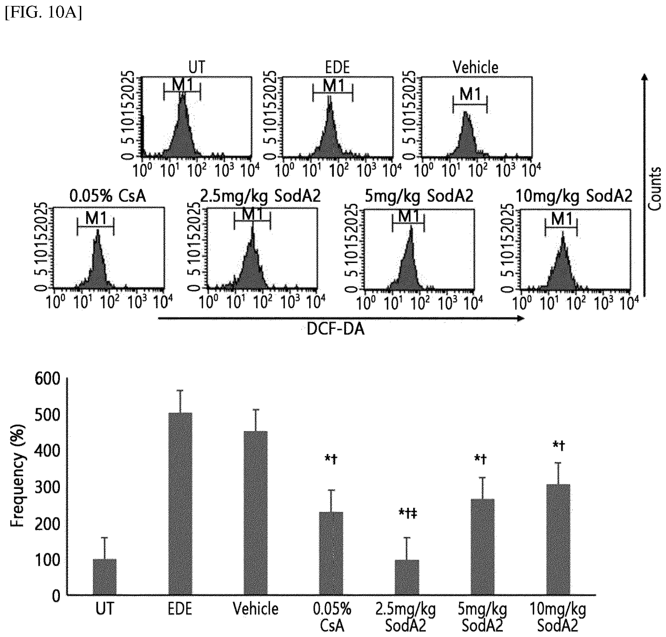

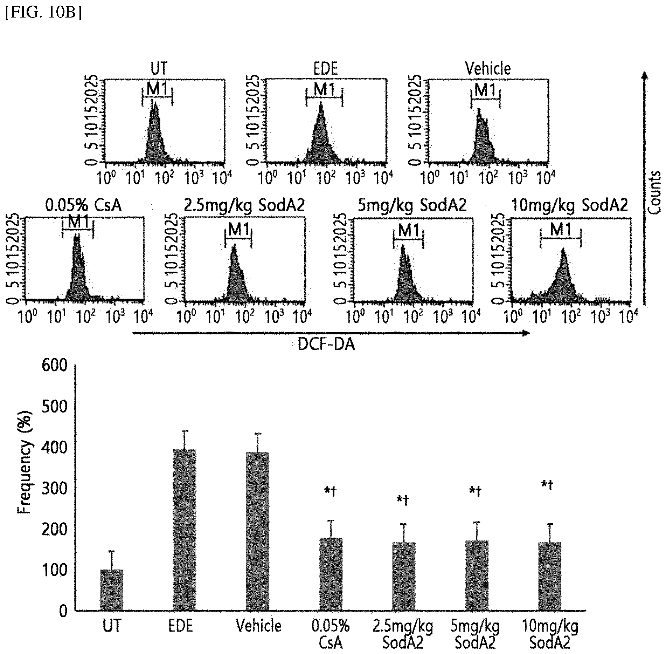

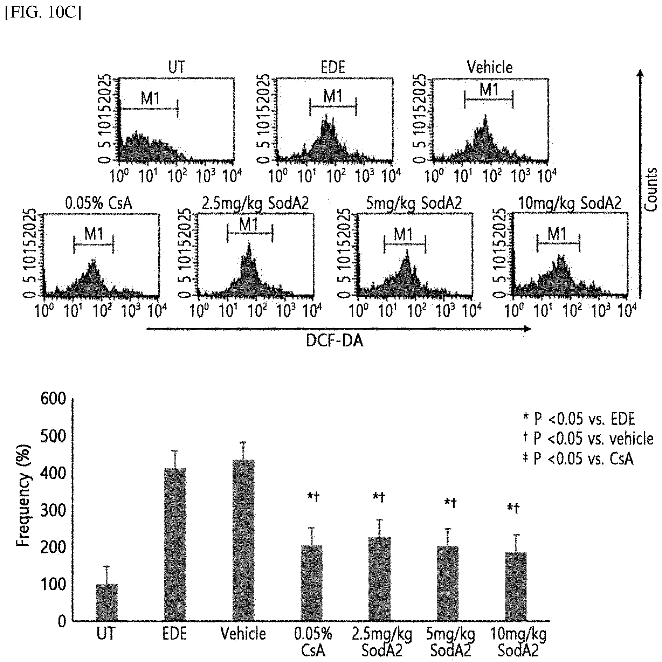

In all of the cornea, conjunctiva, and lacrimal gland, the groups treated with SodA2 showed a similar or better effect of decreasing generation of reactive oxygen species than the group treated with CsA. In particular, in the cornea, the 2.5 mg/kg oral SodA2 group showed a better effect of decreasing generation of reactive oxygen species than the CsA group. These results obtained by measuring the amount of reactive oxygen species generated in the cornea, conjunctiva, and lacrimal gland are shown in FIGS. 10A, 10B, and 10C, respectively.

### Measurement of number of goblet cells in conjunctiva

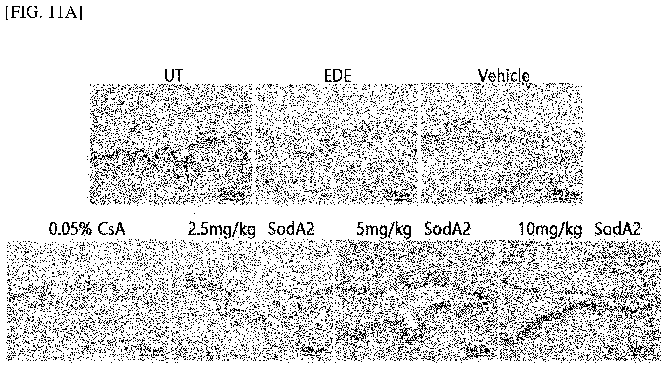

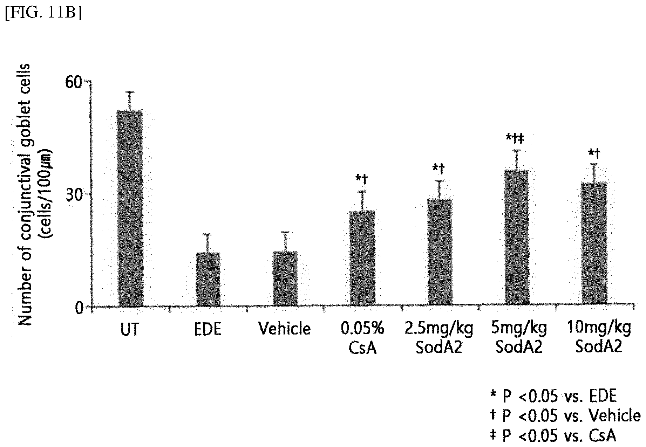

The number of goblet cells in the conjunctiva was 52.1 ± 9.6 cells/100 µm in the UT group, 14.1 ± 3.6 cells/100 µm in the EDE group, and 14.6 ± 2.4 cells/100 µm in the vehicle group. The number of goblet cells was 25.2 ± 3.4 cells/100 µm in the 0.05% CsA group, 27.9 ± 6.9 cells/100 µm in the 2.5 mg/kg oral SodA2 group, 35.8 ± 10.2 cells/100 µm in the 5 mg/kg oral SodA2 group, and 32.2 ± 3.9 cells/100 µm in the 10 mg/kg oral SodA2 group, indicating that there was a significant increase in number of goblet cells in all treatment groups. In particular, the 5 mg/kg oral SodA2 group showed a more significant increase in number of goblet cells than the 0.05% CsA group. These results obtained by measuring the number of goblet cells in the conjunctiva are shown in FIGS. 11A (PAS staining images) and 11B (number of goblet cells).

### Measurement of apoptosis

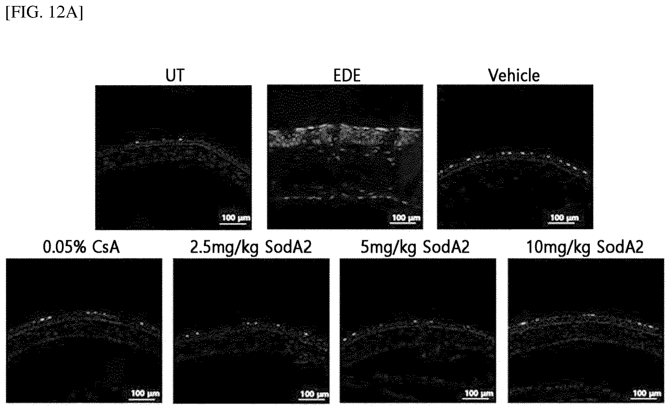

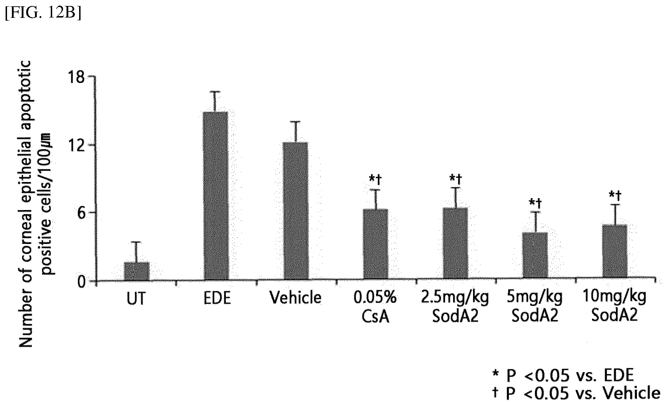

The number of necrotic cells in the cornea was 1.6 ± 0.5 cells/100 µm in the UT group, 14.8 ± 3.3 cells/100 µm in the EDE group, and 12.1 ± 3.0 cells/100 µm in the vehicle group, indicating that the number of necrotic cells significantly increased due to dry eye stimulation. In contrast, the number of necrotic cells was 6.1 ± 2.1 cells/100 µm in the 0.05% CsA group, 6.2 ± 1.1 cells/100 µm in the 2.5 mg/kg oral SodA2 group, 4.0 ± 1.3 cells/100 µm in the 5 mg/kg oral SodA2 group, and 4.6 ± 1.3 cells/100 µm in the 10 mg/kg oral SodA2 group, indicating that the number of dead cells in the cornea was significantly decreased in both groups treated with CsA and oral SodA2 as compared with the EDE and vehicle groups. These results obtained by measuring apoptosis are shown in FIGS. 12A (TUNEL staining images) and 12B (number of apoptotic positive cells).

### Example 4.4. Conclusion

To summarize the above results, oral SodA2 improved tear volume, tear film break-up time, and corneal epithelial damage on the ocular surface in dry eye-induced mice. In addition, oral SodA tended to decrease inflammatory T cells and cytokines in the cornea and conjunctiva. It has also been shown that oral SodA is capable of decreasing oxidative stress in the cornea, conjunctiva, and lacrimal gland, increasing the number of goblet cells in the conjunctiva, and decreasing corneal epithelial apoptosis. Besides, it is determined that oral SodA2 is capable of exhibiting similar or superior efficacy as compared with 0.05% cyclosporin in terms of corneal fluorescent staining score, inflammatory T cells in the cornea, and goblet cell density in the conjunctiva. It is determined that the newly developed oral SodA2 is capable of minimizing ocular surface damage and decreasing inflammation, and thus may be effectively used for treatment of patients with dry eye in the future.

## Claims

1. A pharmaceutical composition for treating or preventing dry eye, comprising a superoxide dismutase (SOD) as an active ingredient.

2. The pharmaceutical composition of claim 1, wherein the composition exhibits one or more selected from increased tear volume, increased tear film break-up time, minimized ocular surface damage, protection of goblet cells in the conjunctiva, decreased number of immune cells or inflammatory cytokines in the cornea or conjunctiva, decreased oxidative stress in the cornea, conjunctiva, or lacrimal gland, and decreased corneal epithelial apoptosis.

3. The pharmaceutical composition of claim 1, wherein the SOD is an isolated or purified enzyme.

4. The pharmaceutical composition of claim 1, wherein the SOD is included in a form of a strain lysate, strain culture, strain culture concentrate, or strain culture extract, or a dried form thereof.

5. The pharmaceutical composition of claim 1, wherein the SOD is an Mn-SOD.

6. The pharmaceutical composition of claim 1, wherein the SOD is a deamidated Mn-SOD.

7. The pharmaceutical composition of claim 1, wherein the SOD is derived from a Bacillus species strain.

8. The pharmaceutical composition of claim 1, wherein the SOD is derived from *Bacillus amyloliquefaciens* GF423 strain (KCTC 13222BP).

9. The pharmaceutical composition of claim 1, wherein amino acid residues 73 and 136 of the SOD are substituted with Asp residues, with respect to SEQ ID NO: 2.

10. The pharmaceutical composition of claim 1, wherein the SOD has an amino acid sequence as set forth in SEQ ID NO: 4.

11. The pharmaceutical composition of claim 1, wherein the dry eye is Sjögren's syndrome or non-Sjögren's syndrome dry eye.

12. The pharmaceutical composition of claim 1, wherein the composition is administered orally.

13. The pharmaceutical composition of claim 12, wherein the SOD in the composition is coated with a coating agent.

14. The pharmaceutical composition of claim 13, wherein the coating agent comprises shellac.

15. A veterinary composition for treating or preventing dry eye, comprising a superoxide dismutase (SOD) as an active ingredient.

16. A food composition for ameliorating or preventing dry eye, comprising a superoxide dismutase (SOD) as an active ingredient.

17. A feed composition for ameliorating or preventing dry eye, comprising a superoxide dismutase (SOD) as an active ingredient.

18. A method for treating or preventing dry eye, comprising administering to a subject the pharmaceutical composition of any one of claims 1 to 14 or a superoxide dismutase (SOD).

19. A use of the pharmaceutical composition of any one of claims 1 to 14 or a superoxide dismutase (SOD), for preventing or treating dry eye.

20. A use of the pharmaceutical composition of any one of claims 1 to 14 or a superoxide dismutase (SOD), in manufacture of a medicament for prevention or treatment of dry eye.
